# EUROPEAN PATENT APPLICATION

(11) **EP 4 814 472 A1**
(43) Date of publication of application: **30.09.2026**
(21) Application number: 25165672.4
(22) Date of filing: 24.03.2025
(51) Int. Cl.: G01N 1/22, C12M 1/00

(54) **LID FOR A DEVICE, PARTICULARLY FOR A MICROBIAL AIR SAMPLER**

(71) Applicant: MBV AG, 8712 Staefa (CH)
(72) Inventor: Roth, Michael, 8712 Stäfa (CH)
(74) Representative: Schulz Junghans Patentanwälte PartGmbB

(57) **Abstract**

The present invention relates to a lid (1), particularly for a device such as a microbial air sampler, wherein the lid (1) comprises: a lower part (10) having a circumferential boundary portion (11) configured to rest on a device (100) having a top surface on which a removable object (e.g. medium plate) (2) is arrangeable, such that the lid (1) covers the object (2), wherein the lower part (10) defines an interior space (12) of the lid configured to accommodate said object, a perforated sieve (20) for the passage of air therethrough such that the air can contact said object (2), when the lid (1) covers the object (2) accommodated in the interior space (12), and an upper part (30) for gripping the lid (1), the upper part (30) being connected to the lower part (10) so that the upper part (30) is arranged opposite the lower part (10) in a first direction (z). According to the invention, the upper part (30) comprises a minimal outer diameter (D1) orthogonal to said first direction (z), and the interior space (12) of the lower part (10) comprises a maximal inner diameter (D2) orthogonal to said first direction (z), wherein said minimal outer diameter (D1) is smaller than said maximal inner diameter (D2).

## Description

The present invention relates to a lid, particularly to a lid for a device such as a microbial air sampler.

The present invention can be particularly applied in the field of particle collection and sampling as well as analysis of particles of all kinds and more, particularly any setup holding a nutrient dish or other dish in similar shape. Especially, it is related to devices for characterization of gas or air quality as necessary in food and beverage industries or clean environments such as cleanrooms and manufacturing environments. Examples for such clean environments are production lines in the pharmaceutical industries, where air quality must be constantly observed and tested.

Microbial air samplers are a specific type of air monitoring devices that focus on the collection of particles on a microbial growth medium - usually in form of a nutrient dish filled with agar media or similar. The dish is placed under a lid with a sieve, essential for the collection of particles to take place. After collection of particles from the gas/air on such a medium plate, the plate is incubated for several hours or even days to let collected living microorganisms grow into visible colonies for subsequent counting and analysis.

There generally exist regulatory limits on how many and which organisms are tolerable in a specific environment and in case limits are exceeded, production lots of pharmaceuticals as one example discarded. Hence analysis of the air quality with a focus on microbial contamination is a critical quality indicator for product release in multiple large industries. Hence, instruments in this field must quantify microbial contamination and gas volume with highest precision and are standardized in ISO 14698-1 and EN17141.

Usually, devices of the afore-mentioned kind comprise a housing with a removable lid, wherein the lid comprises holes (e.g. in form of a sieve) so that the device can suck air into an interior space covered by the lid to allow the air to contact a nutrient medium on a plate that is placed in said interior space. The lid thereby also covers the plate.

The outer diameter of such a lid on a microbial air sampler is principally defined by the size of the largest media plate (typically about 90mm) that is placed on the air sampler and covered by the lid.

However, a lid with such a large diameter is difficult to handle in particularly for a small hand, considering that the lid should be held from the side to prevent placing any part of the hand above the sieve, which would increase the risk of contaminating the lid and thereby the sampling that follows.

The heavier the lid, the more important is a good grip of the lid. E.g. in case of lids partially or fully manufactured from stainless steel, which is the preferred material in clean room environments but considerable heavier than the aluminum that is often used for air samplers currently on the market maintaining a good grip on the lid during handling of the lid is very important.

Furthermore, the more force it takes to loosen a locking mechanism of the lid by means of which the lid is held on the device, the more important is a good grip of the lid, too. For instance, in case of a bayonet locking mechanism that is most often used for air samplers currently on the market, the lid may be difficult to open for a small hand, especially if the lid has been closed fast and hard. This also applies to magnetic closing mechanism that ensure that even a heavy lid holds well on the device and to the friction forces caused by polymer seal rings that can be placed between device and lid.

Further, the smoother the surface, the more important it is to ensure a good grip, too. For instance, an electropolished stainless steel surface, which is the preferred surface in clean room environments can be much more slippery during handling than a rough surface that is however undesirable in clean room environments as they are more difficult to clean and therefore increase the risk of microbial contamination of the surface.

Finally, geometries that are suitable for manual handling might not necessarily be suitable for robotic handling and vice versa.

Based on the above, the problem to be solved by the present invention is to provide a lid with improved handling capabilities so that the lid can also be handled by a smaller hand, particularly without impeding robotic handling of the lid.

The above-stated problem underlying the present invention is solved by a lid having the features of claim 1. Further aspects of the present invention relate to a microbial air sampler, comprising a lid according to the present invention and to an arrangement.

Preferred embodiments of these aspects of the present invention are stated in the corresponding dependent claims and are also described below.

According to claim 1 a lid is disclosed, comprising
- a lower part having a circumferential boundary portion configured to rest on a device, particularly in form of a microbial air sampler, the device having a top surface on which a removable object is placed, such that the lid covers the object when the lid rests on the device, wherein the lower part defines an interior space of the lid configured to accommodate said object when the lid rests on the device,
- a sieve for the passage of air therethrough such that the air can contact said object, when the lid covers the object accommodated in the interior space, and
- an upper part for gripping the lid, the upper part being connected to the lower part so that the upper part is arranged opposite the lower part in a first direction,
wherein the upper part comprises a minimal outer diameter orthogonal to said first direction, and the interior space of the lower part comprises a maximal inner diameter orthogonal to said first direction, wherein said minimal outer diameter is smaller than said maximal inner diameter.

Particularly, the sieve (e.g. a perforated plate) comprises a plurality of openings so that air can flow through these openings of the sieve, wherein said openings can be circular through-holes or slots or any other suitable opening allowing passage of air through the sieve. Generally, also other fluids (particularly gases) than air or mixtures of air with other fluids (particularly gases) can be processed with the lid or microbial air sampler according to the present invention.

Particularly, during operation of the device with the lid being placed on the device, the first direction typically corresponds to the vertical. However, such a device may also be operated in a different orientation (e.g. in case of a portable device). The first direction may then also point in another direction and may even correspond to the horizontal.

Due to the smaller outer diameter of the upper part of the lid compared to the lower part of the lid, the handling and holding of the lid is improved for smaller hands and fingers of users of the lid while still allowing to e.g. accommodate the object, particularly a medium plate in the lower part of the lid.

With respect to grabbing/holding the lid at the minimal outer diameter, even small hands can reach around 180° of the minimal outer diameter (upper part) with at least two fingers in case of objects such as medium plates typically used with devices such as microbial air samplers. This also prevents the hand of the user from slipping off on a smooth/slippery outer surface of the upper part of the lid.

Due to the smaller minimal outer diameter of the upper part compared to the lower part of the lid, a reduced strain on the fingers of the user can be achieved which is less tiring to the hand of the user (as compared to having to grab the lid tightly to create sufficient friction).

Furthermore, due to the invention, removing the lid from the corresponding device, particularly microbial air sampler, can be performed more reliably with one hand. It is not necessary to use the second hand e.g. to hold/stabilize the device. The second hand is free to manipulate the object/medium plate.

The geometry of the upper part of the lid cannot only be optimized for manual handling but also for robotic handling.

Particularly, the lid according to the present invention can be used with different devices that require the lid to cover an object along which an air flow shall be passed. Particularly, such an object typically has an outer diameter orthogonal to the first direction in the range from 50 mm to 95mm, particularly 55 mm to 90 mm.

According to a preferred embodiment, the object is a medium plate for collecting microbial and/or particulate matter transported by an air flow flowing through the sieve and along a medium carried by the medium plate (e.g. agar plate). Preferably, the corresponding device is a microbial air sampler. Particularly, when the object is a medium plate, the first direction can run orthogonal to the medium plate, particularly orthogonal to a bottom of the medium plate. Particularly, a medium plate used with the device, particularly microbial air sampler, typically has an outer diameter orthogonal to the first direction in the range from 50 mm to 95mm, particularly 55 mm to 90 mm.

According to a preferred embodiment of the lid, the upper part comprises a circumferential lateral wall that encloses an air intake of the lid, the air intake comprising a bottom formed by the sieve that is in fluid communication with the air intake so that an airflow can pass through the air intake and the sieve into the interior space of the lower part of the lid. The sieve can be integrally formed with the lid or permanently attached thereto. Particularly, the sieve can be integrally formed with the lower part of the lid or attached thereto. Alternatively, the sieve can be a separate part designed to be releasable from the remainder of the lid.

Furthermore, in a preferred embodiment of the present invention, the sieve separates the air intake from the interior space of the lower part of the lid.

Further, particularly, the air intake can accommodate an air column that is entering the interior space through the sieve. Particularly, the first direction corresponds to an inflow direction along which the airflow / air column enters the air intake and flows through the sieve.

In yet another preferred embodiment of the lid, the minimal outer diameter is smaller than a diameter of the sieve orthogonal to the first direction.

Further, according to a preferred embodiment, said circumferential lateral wall of the upper part of the lid comprises said minimal outer diameter.

Further, according to a preferred embodiment of the lid, the minimal outer diameter is in the range from 98.0 mm to 30.0 mm, preferably in the range from 95 mm to 30 mm preferably in the range from 85 mm to 35 mm, more preferably in the range from 80 mm to 35 mm.

Further, according to a preferred embodiment of the lid, the maximal inner diameter of the interior is in the range from 120 mm to 60 mm, preferably in the range from 115 mm to 70 mm, more preferably in the range from 110 mm to 95 mm.

Further, according to a preferred embodiment of the lid, the upper part of the lid comprises a rim, formed as a circumferential protrusion of the circumferential lateral wall, said protrusion extending outward at an upper end of the circumferential lateral wall, wherein particularly the rim comprises an outer diameter orthogonal to the first direction, the outer diameter of the rim being larger than the minimal outer diameter.

Particularly, the rim serves to prevent the lid from slipping out from between the fingers when the upper part of the lid is gripped by the hand of a user. In case a robotic gripper is used, the rim prevents the lid from slipping out of the robotic gripper by having the rim rest on the gripper.

In a preferred embodiment of the lid, said circumferential lateral wall of the upper part of the lid is cylindrical, particularly circular cylindrical, wherein particularly said minimal outer diameter is a constant outer diameter of the circumferential lateral wall.

According to yet another preferred embodiment of the lid, said circumferential lateral wall of the upper part of the lid comprises a conical shape.

Further, according to a preferred embodiment of the lid, the circumferential lateral wall of the upper part of the lid comprises a lower portion and an upper portion adjacent the lower portion, wherein the lower portion is arranged between the upper portion and the lower part of the lid with respect to the first direction, and wherein the lower portion comprises said minimal outer diameter, and wherein the upper portion comprises an outer diameter orthogonal to the first direction that is larger than the minimal outer diameter. Particularly, in the lower portion, the air intake can have an inner diameter that is smaller than an inner diameter of the air intake in the upper portion of the circumferential wall.

Further, according to a preferred embodiment of the lid, an outer surface of the circumferential lateral wall of the upper part of the lid comprises a non-circular cross-sectional contour orthogonal to the first direction. In a preferred embodiment of the lid, the cross-sectional contour is a polygon. Particularly, the polygon can be a hexagon, an octagon, a decagon, or a dodecagon. The polygon can also have more than twelve sides. A polygonal shape is advantageous as the edges between adjacent sides of the polygon provide additional grip.

Further, according to a preferred embodiment of the lid, an outer surface of the circumferential lateral wall comprises at least one or multiple recesses, wherein particularly the respective recess is configured to receive a finger of a person gripping the upper part.

Particularly, the respective recesses can extend in the first direction. The respective recess can be a groove having a concave shape.

Further, according to a preferred embodiment of the lid, the circumferential lateral wall comprises a height in the first direction of at least 5 mm, particularly at least 10 mm, particularly at least 15 mm. Preferably, the height is in the range from 5 mm to 50 mm preferably in the range from 10 to 50 mm, more preferably in the range from 15 mm to 40 mm.

Further, according to a preferred embodiment of the lid, the upper part is configured to accommodate a gripper of a robotic device.

According to yet another aspect of the present invention, a microbial air sampler is disclosed, comprising a top surface for supporting a medium plate and a lid according to the invention (wherein the object is a medium plate) for covering the medium plate, wherein the microbial air sampler is configured to suck an airflow through the sieve when the lid covers the medium plate being arranged on said top surface so that microbial and/or particulate matter transported by the airflow is collected on a medium carried by the medium plate.

According to a further aspect of the present invention, an arrangement comprising a lid according to the present invention and a medium plate is disclosed, wherein the minimal outer diameter of the upper part of the lid is smaller than an outer diameter of the medium plate.

In the following, embodiments of the present invention as well further features and advantages and other aspects of the present invention shall be described with reference to the Figures, wherein
- Figs. 1A-C: show a perspective view of an embodiment of the lid according to the present invention (A) as well as cross sectional views (B, C) of the lid, wherein the upper portion of the lid comprises a rim;
- Figs. 2A-C: show a perspective view of an embodiment of the lid according to the present invention (A) as well as cross sectional views (B, C) of the lid, wherein compared to Fig. 1 the upper portion of the lid is free of a rim;
- Figs. 3A-C: show a perspective view of an embodiment of the lid according to the present invention (A) as well as cross sectional views (B, C) of the lid, wherein the minimal outer diameter of the upper portion of the lid is smaller than a diameter of the sieve and wherein the upper part of the lid comprises a rim;
- Figs. 4A-C: show a perspective view of an embodiment of the lid according to the present invention (A) as well as cross sectional views (B, C) of the lid, wherein the minimal outer diameter of the upper portion of the lid is smaller than a diameter of the sieve and wherein the upper part of the lid is free of a rim;
- Figs. 5A-C: show a perspective view of an embodiment of the lid according to the present invention (A) as well as cross sectional views (B, C) of the lid, wherein the minimal outer diameter of the upper portion of the lid is located at a lower portion of the upper part and the upper part comprises an upper portion having an outer diameter being larger than said minimal outer diameter, so that particularly the lid comprises an hourglass-like shape;
- Figs. 6A-C: show a perspective view of an embodiment of the lid according to the present invention (A) as well as cross sectional views (B, C) of the lid, wherein the upper portion of the lid comprises multiple recesses for accommodating fingers of a user grabbing the upper part; and
- Figs. 7A-B: show a perspective view of a microbial air sampler comprising a lid according to the present invention (A) and a side view (B) of the microbial air sampler.

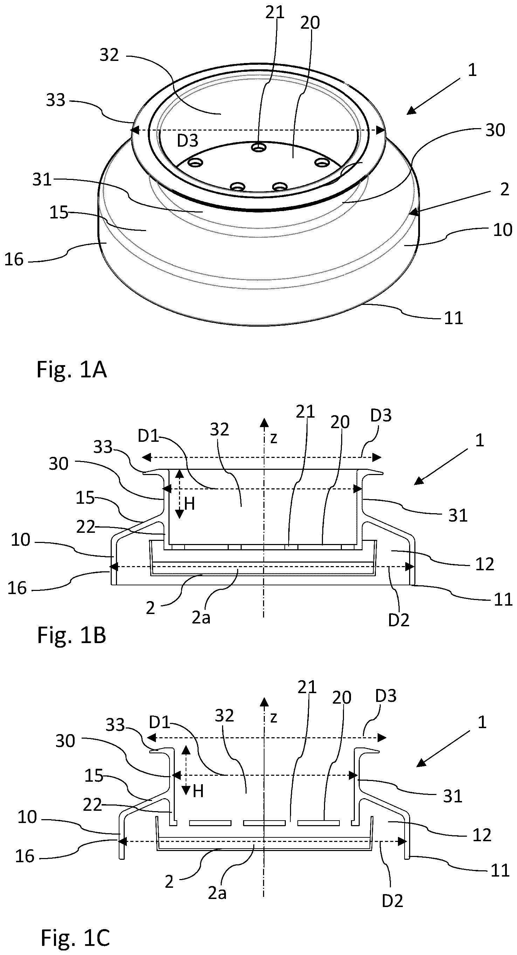

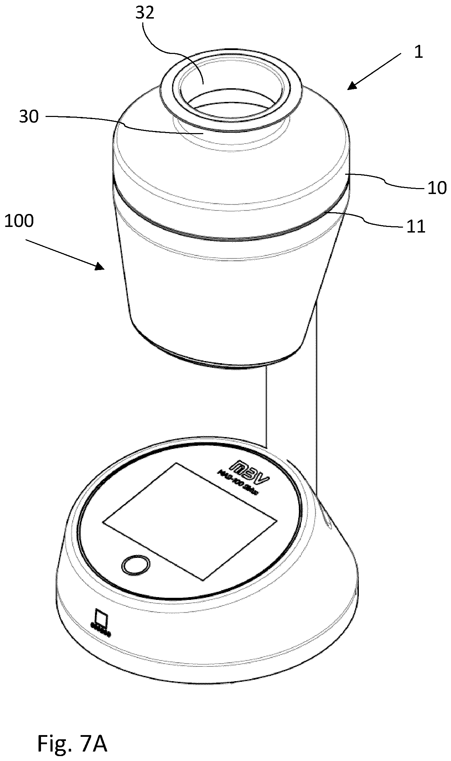

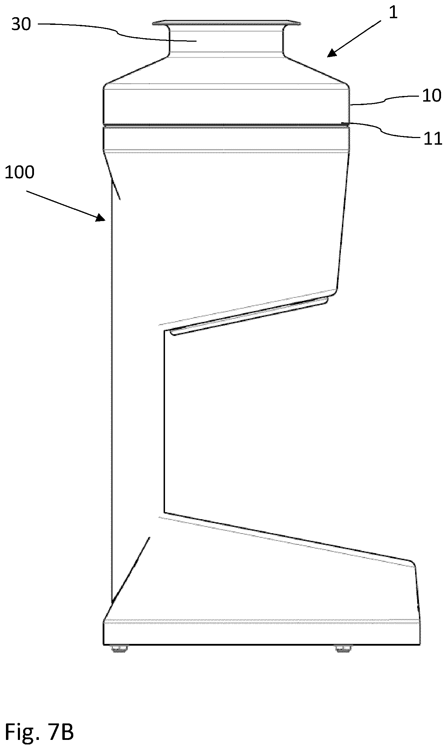

Fig. 1A shows in conjunction with Figs. 1B-C an embodiment of a lid 1 according to the present invention. The lid 1 is configured to cover an object 2 placed on a top side of a device 100. The lid 1 comprises a sieve 20 allowing the passage of an airflow therethrough to flow along the object 2. The sieve 20 comprises through-holes 21 that allow the air to pass through. Particularly, the object 2 can be a medium plate 2 carrying a medium 2a, particularly a nutrient medium. Furthermore, particularly, the device 100 can be an (e.g. portable) microbial air sampler 100 as shown in Figs. 7A-B that is configured to suck the airflow through the sieve 20 when the lid 1 covers the medium plate 2 being arranged on said top surface of the microbial air sampler 100 so that microbial and/or particulate matter transported by the airflow is collected on the medium 2a carried by the medium plate 2. After said sampling, the medium plate 2 can be incubated under appropriate conditions to allow microbial growth to assess microbial contamination levels in the measured air.

In order to operate such devices 100, the respective lid 1 has to be e.g. manually lifted to place a medium plate 2 onto the top surface of the device 100 or to remove a medium plate 2 from the device 100 to replace it with a new one. The present invention now suggests lids 1 that are especially suited to be grabbed manually in a secure fashion even in case the respective user comprises relatively small hands. This is accomplished by configuring an upper part 30 of the lid 1 with a reduced outer diameter as will be described in the following.

According to the embodiment shown in Figs. 1A-C, the lid 1 comprises a lower part 10 having a circumferential boundary portion 11 configured to rest on the device 100 to cover the medium plate 2 arranged on the top surface of the microbial air sampler 100, such that the lid 1 covers the medium plate 2, wherein the lower part 10 defines an interior space 12 of the lid 1 configured to accommodate the medium plate 2 as shown in Figs. 1B-C. Further, the lid 1 comprises a sieve 20 for the passage of said airflow therethrough such that the air can contact said the medium 2a carried by the medium plate 2, when the lid 1 covers the medium plate 2 and medium 2a contained therein to accommodate them in the interior space 12 of the lid 1. Further, the lid 1 comprises said upper part 30 for gripping the lid 1, the upper part 30 being connected to the lower part 10 so that the upper part 30 is arranged opposite the lower part 10 in a first direction z. According to the invention, the upper part 30 comprises a minimal outer diameter D1 orthogonal to said first direction z, and the interior space 12 of the lower part 10 comprises a maximal inner diameter D2 orthogonal to said first direction z, wherein said minimal outer diameter D1 is smaller than said maximal inner diameter D2. This allows the user to conveniently grab the lid 1 by the upper part 30.

Particularly, the upper part 30 of the lid 1 comprises a circumferential lateral wall 31 that encloses an air intake 32 of the lid 1, the air intake 32 comprising a bottom formed by the sieve 20 via which the air intake 32 is in fluid communication with the interior space 12 defined by the lower part 10 of the lid. Particularly, as indicated in Figs. 1B-C, said circumferential lateral wall 31 comprises said minimal outer diameter D1.

Furthermore, to further increase handleability of the lid 1, the upper part 30 of the lid 1 can comprises a rim 33 as shown in Fig. 1A-C formed as a circumferential protrusion of the circumferential lateral wall 31, said protrusion extending outward at an upper end of the circumferential lateral wall 31, wherein particularly the rim 33 comprises an outer diameter D3 orthogonal to the first direction z, wherein the outer diameter D3 of the rim 33 is larger than the minimal outer diameter D1. Particularly, the rim 33 prevents the lid 1 from slipping out from between the fingers when the upper part 30 of the lid 1 is gripped by the hand of the user.

Due to the minimal outer diameter D1 of the upper part 30 / circumferential lateral wall 31 being smaller than the maximal inner diameter D2 of the lower part 10, the lid 1 particularly comprises a circumferential step 15 forming a top wall portion of the interior space 12 of the lower part 10 of the lid 1.

Furthermore, the circumferential lateral wall 31 of the upper part 30 of the lid 1 merges into a circumferential wall portion 22 of the lower part 10 of the lid 1, which circumferential wall portion 22 connects to the sieve 20 at a lower end of said circumferential wall portion 22. Particularly, the circumferential wall portion 22 together with the sieve 20 protrudes into the interior space 12 of the lower part 10 of the lid 1, which allows the perorated sieve 20 to protrude into the medium plate 2 when the lid 2 covers the medium plate 2 as intended. This improves contact between the medium 2a and the airflow passing along the air intake 2 through the sieve 20.

Furthermore, the lower part 10 of the lid 1 comprises a circumferential lateral wall 16 connected to the top wall portion 15, wherein the circumferential lateral wall 16 of the lower part 10 encloses the medium plate 2 when the lid 1 covers the medium plate 2 as intended. Particularly, the maximal inner diameter D2 is the maximal inner diameter of this circumferential lateral wall 16 of the lower part 10 of the lid. 1

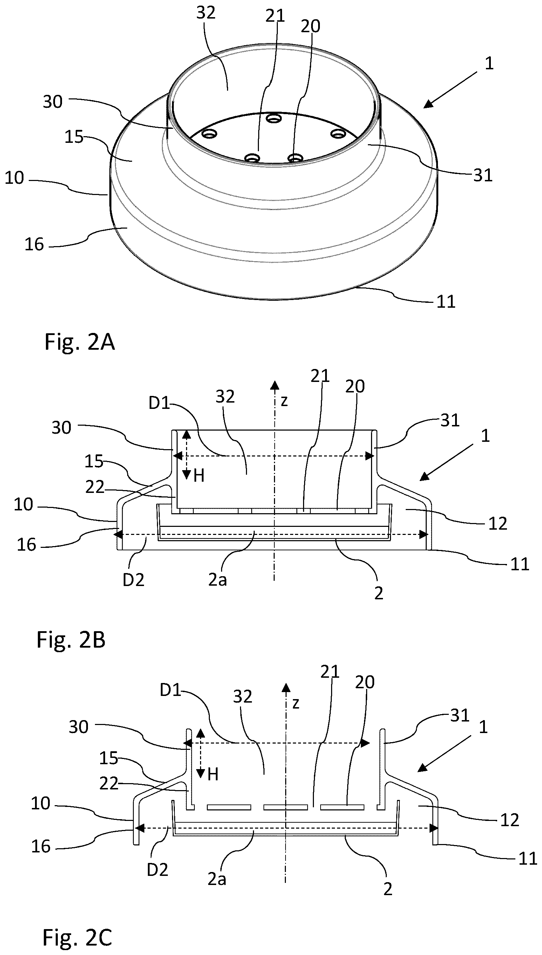

Figs. 2A-C show a further embodiment of the lid 1 that corresponds to the embodiment of the lid 1 shown in Fig. 1A-C with the difference that the lid 1 shown in Figs. 2A-C is free of a rim 33. Particularly, the circumferential lateral wall 31 of the upper part 30 of the lid 1 can be circular cylindrical.

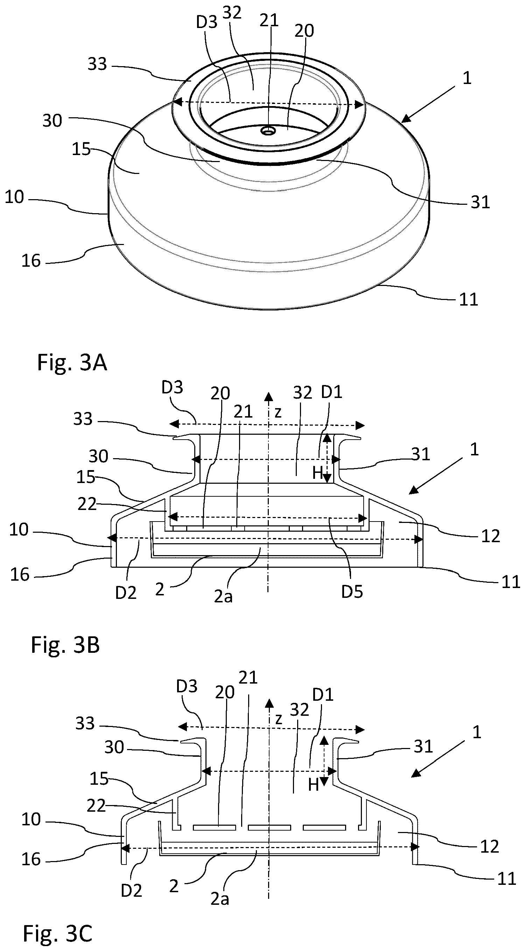

Figs. 3A-C show a further embodiment of the lid 1 according to the present invention, that corresponds to a modification of the embodiment shown in Fig. 1A-C. However, in the embodiment shown in Figs. 3A-C, the minimal outer diameter D1 of the upper part is further reduced. This can be achieved by joining the circumferential wall portion 22 that connects to the sieve 20 to the top wall / step 15 of the lower part 10. This achieves that the minimal outer diameter D1 is smaller than a diameter D5 of the sieve 20 which in turn is preferably smaller than the outer diameter of the medium plate 2.

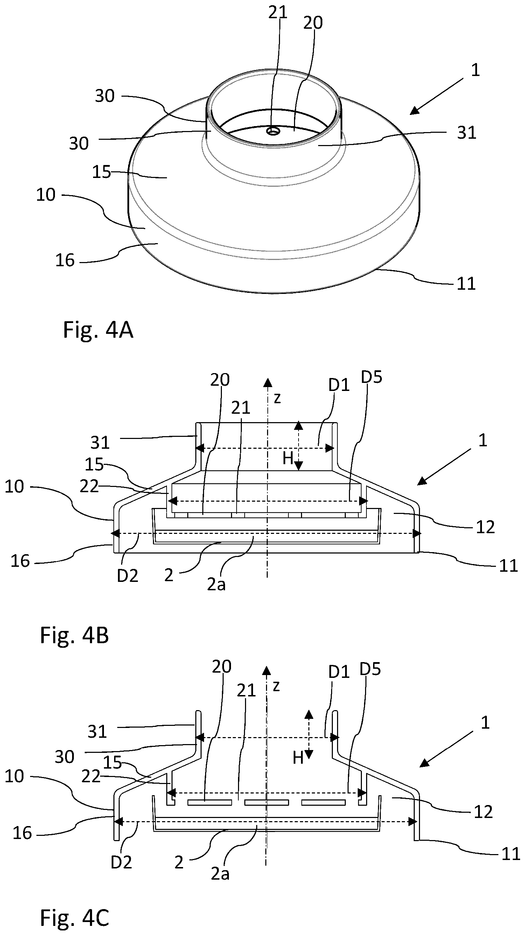

Furthermore, Figs. 4A-C show a further embodiment of the lid 1 that corresponds to the embodiment of the lid 1 shown in Figs. 3A-C with the difference that the lid 1 shown in Fig. 4A-C is free of a rim 33. Particularly, the circumferential lateral wall 31 of the upper part 30 of the lid 1 can be circular cylindrical.

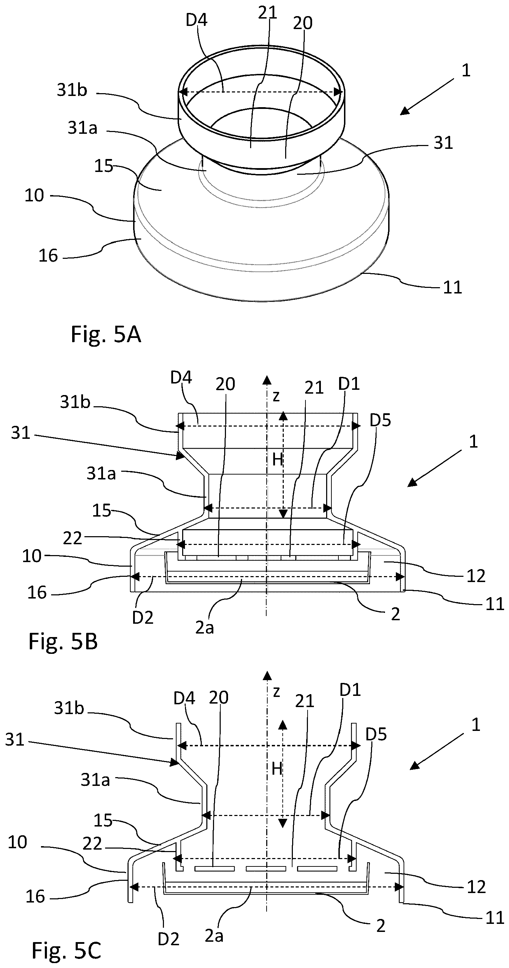

Figs. 5A-C show a further embodiment of the lid 1 according to the present invention, wherein the circumferential lateral wall 31 comprises a lower portion 31a and an upper portion 31b connected to the lower portion 31a, wherein the lower portion 31a is arranged between the upper portion 31b and the lower part 10 of the lid 1 with respect to the first direction z, and wherein the lower portion 31a comprises said minimal outer diameter D1, and wherein the upper portion 30 comprises an outer diameter D4 orthogonal to the first direction z that is larger than the minimal outer diameter D1. Thus. the upper part 30 comprises a circumferential constriction formed by the lower portion 31a. The lower portion 31a merges into the upper portion 31b which can expand outwards in a conical fashion to connect to a cylindrical extension. Due to the constriction the lid can assume an hourglass-like shape that allows to conveniently grab the upper part (e.g. from above placing the finger tips into the constriction).

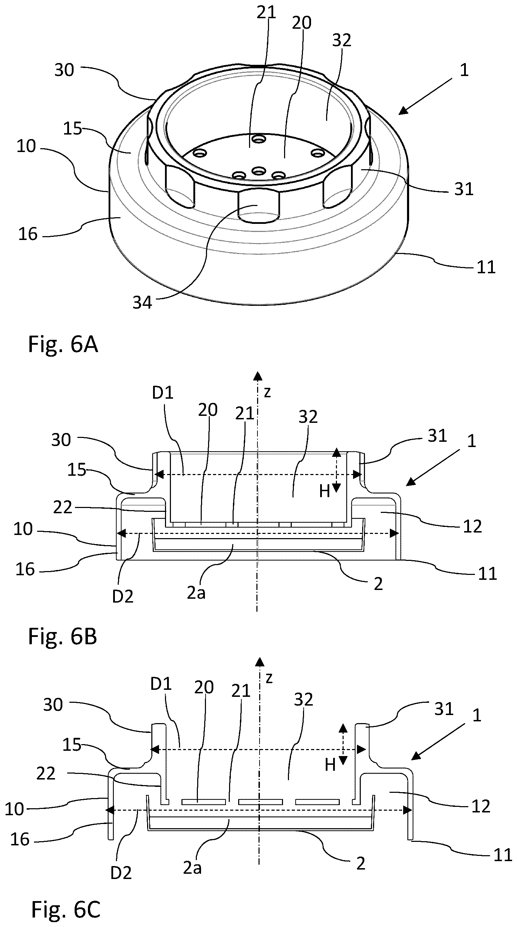

Finally, Figs. 6A-6C show an embodiment of the lid 1, wherein the circumferential lateral wall 31 of the upper part comprises an outer surface having a plurality of recesses 34 that may extend along the first direction z and may have a concave profile. In this way, the recesses 34 are configured to accommodate fingers of a user grabbing the lid 1 from above.

## Claims

1. A lid (1), comprising:
- a lower part (10) having a circumferential boundary portion (11) configured to rest on a device (100) having a top surface on which a removable object (2) is arrangeable, such that the lid (1) covers the object (2), wherein the lower part (10) defines an interior space (12) of the lid configured to accommodate said object,
- a sieve (20) for the passage of air therethrough such that the air can contact said object (2), when the lid (1) covers the object (2) accommodated in the interior space (12),
- an upper part (30) for gripping the lid (1), the upper part (30) being connected to the lower part (10) so that the upper part (30) is arranged opposite the lower part (10) in a first direction (z)
**characterized in that**
the upper part (30) comprises a minimal outer diameter (D1) orthogonal to said first direction (z), and the interior space (12) of the lower part (10) comprises a maximal inner diameter (D2) orthogonal to said first direction (z), wherein said minimal outer diameter (D1) is smaller than said maximal inner diameter (D2).

2. The lid according to claim 1, wherein the upper part (30) comprises a circumferential lateral wall (31) that encloses an air intake (32) of the lid (1), the air intake (32) comprising a bottom formed by the sieve (20).

3. The lid according to claim 2, wherein said circumferential lateral wall (31) comprises said minimal outer diameter (D1),

4. The lid according to one of the preceding claims, wherein the minimal outer diameter (D1) is in the range from 98.0 mm to 30.0 mm, preferably in the range from 95 mm to 30 mm, more preferably in the range from 85 mm to 35 mm, more preferably in the range from 80 mm to 35 mm.

5. The lid according to one of the claims 2 to 4, wherein the upper part (30) of the lid (1) comprises a rim (33) formed as a circumferential protrusion of the circumferential lateral wall (31), said protrusion extending outward at an upper end of the circumferential lateral wall (31), wherein particularly the rim (33) comprises an outer diameter (D3) orthogonal to the first direction (z), the outer diameter (D3) of the rim (33) being larger than the minimal outer diameter (D1).

6. The lid according to one of the claims 2 to 5, wherein said circumferential lateral wall (31) is cylindrical, particularly circular cylindrical, wherein particularly said minimal outer diameter (D1) is a constant outer diameter of the circumferential lateral wall (31).

7. The lid according to one of the claims 2 to 5, wherein said circumferential lateral wall (31) comprises a conical shape.

8. The lid according to one of the claims 2 to 5, wherein the circumferential lateral wall (31) comprises a lower portion (31a) and an upper portion (31b) connected to the lower portion (31a), wherein the lower portion (31a) is arranged between the upper portion (31b) and the lower part (10) of the lid (1) with respect to the first direction (z), and wherein the lower portion (31a) comprises said minimal outer diameter (D1), and wherein the upper portion (30) comprises an outer diameter (D4) orthogonal to the first direction (z) that is larger than the minimal outer diameter (D1).

9. The lid according to one of the preceding claims, wherein an outer surface of the circumferential lateral wall (31) comprises a non-circular cross-sectional contour orthogonal to the first direction (z).

10. The lid according to claim 9, wherein the cross-sectional contour is a polygon.

11. The lid according to one of the preceding claims, wherein an outer surface of the circumferential lateral wall (31) comprises at least one or multiple recesses (34), wherein particularly the respective recess (34) is configured to receive a finger of a person gripping the upper part (30).

12. The lid according to one of the claims 2 to 11, wherein the circumferential lateral wall (31) comprises a height (H) of at least 10 mm.

13. The lid according to one of the preceding claims, wherein the upper part (30) is configured to accommodate a gripper of a robotic device.

14. A microbial air sampler (100), comprising a top surface for supporting a medium plate (2) and a lid (1) according to one of the preceding claims for covering the medium plate (2), wherein the microbial air sampler (100) is configured to suck an air flow through the sieve (20) when the lid (1) covers the medium plate (2) being arranged on said top surface so that microbial and/or particulate matter transported by the airflow is collected on a medium (2a) carried by the medium plate (2).
